# EUROPEAN PATENT APPLICATION

(11) **EP 2 705 835 A1**
(43) Date of publication of application: **12.03.2014**
(21) Application number: 12290189.5
(22) Date of filing: 08.06.2012
(51) Int. Cl.: A61K 9/00, A61K 38/12, A61K 47/10

(54) **Aqueous gelling compositions of soluble active pharmaceutical peptides providing modified release**

(71) Applicant: IPSEN PHARMA S.A.S., 91200 Boulogne-Billancourt (FR)
(72) Inventor: Baronnet, Marie-Madeleine, 27530 Ezy sur Eure (FR); Richard, Joël, 78490 Mere (FR); Mondoly, Nathalie, 78150 Le Chesnay (FR); Nourisson, Didier, 28500 Vernouillet (FR)
(74) Representative: Audonnet, Nathalie

(57) **Abstract**

The present invention relates to an aqueous pharmaceutical composition comprising a peptide as the active ingredient, and one or more water-soluble or water-dispersible gelling agents.

## Description

The present invention relates to an aqueous pharmaceutical composition comprising one or more water-soluble or water-dispersible gelling agents and a peptide as the active substance, in particular a peptide as melanocortin receptor ligand.

Melanocortins are a family of regulatory peptides which are formed by post-translational processing of pro-hormone pro-opiomelanocortin. Melanocortins have been found in a wide variety of normal human tissues including the brain, adrenal, skin, testis, spleen, kidney, ovary, lung, thyroid, liver, colon, small intestine and pancreas. Melanocortin peptides have been shown to exhibit a wide variety of physiological activities including the control of behavior and memory, affecting neurotrophic and antipyretic properties, as well as affecting the modulation of the immune system, the control of the cardiovascular system, analgesia, thermoregulation and the release of other neurohumoral agents including prolactin, luteinizing hormone and biogenic amines. Five melanocortin receptors (MC-R) have been characterized to date: melanocyte-specific receptor (MC1-R), corticoadrenal-specific ACTH receptor (MC2-R), melacortin-3 (MC3-R), melanocortin-4 (MC4-R) and melanocortin-5 receptor (MC5-R). There has been great interest in melanocortin (MC-R) receptors as targets for the design of novel therapeutics to treat disorders of body weight such as obesity and cachexia. Both genetic and pharmacological evidence points toward central MC4-R receptors as the principal target. The current progress with receptor-selective agonists and antagonists evidences the therapeutic potential of melanocortin receptor activation, particularly MC4-R. Due to this therapeutic potential, there is a need of new formulations for this type of compounds, in particular a need of injection formulations.

Parenteral injection of a soluble active pharmaceutical ingredient in saline classically leads to a high value of the drug plasma peak concentration (Cₘₐₓ) and an initial high variation rate of the plasmatic drug concentration that results in a short time (Tₘₐₓ) to reach the maximal concentration Cₘₐₓ, i.e. the burst effect. These two features of the pharmacokinetic (PK) profile can induce side effects, which may jeopardize the development and use of the drug.

There is thus a real need to develop a pharmaceutical composition that modifies the PK profile reducing Cₘₐₓ and increasing Tₘₐₓ, aiming to give a smooth immediate release, to reduce side effects and to increase the tolerance of the treatment.

The Applicant has now discovered that the introduction of certain gelling agents into a particular pharmaceutical composition can satisfy the abovementioned objectives.

In particular, it has discovered that by introducing at least one water-soluble or water-dispersible gelling agent into a pharmaceutical composition that comprises a particular peptide, a pharmaceutical composition with a reduced Cₘₐₓ can be obtained.

The injection of such a composition is quick and simple, does not cause any pain and does not require any particular know-how.

Furthermore, the injection of this composition makes it possible to obtain satisfactory physical properties, in particular in terms of solubility and filterability.

In a particularly advantageous manner, the Applicant has also found that the composition according to the invention ensures a peptide chemical stability.

In addition, the treatment using such a composition permits to modify the *in vitro* peptide release.

Finally, the composition according to the present invention allows a sustained-release of the active ingredient over at least 2 hours.

One subject of the present invention is thus an aqueous pharmaceutical composition comprising a peptide as the active substance and one or more water-soluble or water-dispersible gelling agents.

Other features, aspects, subjects and advantages of the present invention will emerge more clearly on reading the description and the examples which follow.

In the text herein below, unless otherwise indicated, the limits of a range of values are included in that range, especially in the expression "ranging from".

In the text herein below, the term "at least one" is equivalent to "one or more".

Unless otherwise indicated, the following definitions are set forth to illustrate and define the meaning and scope of the various terms used to describe the invention herein.

The term "peptide" is understood to mean a peptide containing up to 50 amino acids and /or with a molecular weight up to about 6,000 Da (6,000 ± 200 Da).

The term "water-soluble" gelling agent is understood to mean that the gelling agent used in the composition according to the present invention is soluble in water. Preferably, the gelling agent has solubility in water measured at 25° C higher than 10 mg/mL, and preferably higher than 30 mg/mL.

The term "water-dispersible" gelling agent is understood to mean that the gelling agent used in the composition according to the present invention is miscible or can be dispersed in water at a concentration measured at 25° C higher than 10 mg/mL, and preferably higher than 30 mg/mL.

The term "gelling agent" defines a compound that can be solubilized, dispersed or mixed with the pharmaceutical composition to modify its rheological behaviour, more particularly its viscosity, and can lead to a higher viscosity composition or the formation of a hydrogel. The term "gelling agent" means a gelling agent or a mixture of gelling agents.

The active ingredient of the pharmaceutical composition of the present invention is a peptide.

Preferably, the peptide is selected from a ligand of one or more of the melanocortin receptors (MC-R). The melanocortin receptor may be selected from melanocyte-specific receptor (MC1-R), corticoadrenal-specific ACTH receptor (MC2-R), melacortin-3 (MC3-R), melanocortin-4 (MC4-R) and melanocortin-5 receptor (MC5-R).

The active ingredient of the drug of the composition of the present invention may be selected from those described in the PCT applications WO 2007/008704 or WO 2008/147556.

In a preferred embodiment, the peptide is a ligand of melanocortin MC4 receptor.

In a preferred embodiment, the peptide is a compound of formula (I):

(R²R³)-A¹-c (A²-A³-A⁴-A⁵-A⁶-A⁷-A⁸-A⁹)-A¹⁰-R¹ (I)

wherein:
A¹ is Acc, HN-(CH₂)ₘ-C(O), L- or D-amino acid or deleted;
A² is Cys, D-Cys, hCys, D-hCys, Pen, D-Pen, Asp or Glu;
A³ is Gly, Ala, β-Ala, Gaba, Aib, D-amino acid or deleted;
A⁴ is His, 2-Pal, 3-Pal, 4-Pal, Taz, 2-Thi, 3-Thi or (X¹,X²,X³,X⁴,X⁵)Phe;
A⁵ is D-Phe, D-1-Nal, D-2-Nal, D-Trp, D-Bal, D-(X¹,X²,X³,X⁴,X⁵)Phe, L-Phe or D-(Et)Tyr;
A⁶ is Arg, hArg, Dab, Dap, Lys, Orn or HN-CH((CH₂)ₙ-N(R⁴R⁵))-C(O);
A⁷ is Trp, 1-Nal, 2-Nal, Bal, Bip, D-Trp, D-1-Nal, D-2-Nal, D-Bal or D-Bip;
A⁸ is Gly, D-Ala, Acc, Ala, β-Ala, Gaba, Apn, Ahx, Aha, HN-(CH₂)ₛ-C(O) or deleted;
A⁹ is Cys, D-Cys, hCys, D-hCys, Pen, D-Pen, Dab, Dap, Orn or Lys;
A¹⁰ is Acc, HN-(CH₂)ₜ-C(O), L- or D-amino acid or deleted;
R¹ is -OH or -NH₂;
R² and R³ is, independently for each occurrence, H, (C₁-C₃₀)alkyl, (C₁-C₃₀)heteroalkyl, (C₁-C₃₀)acyl, (C₂-C₃₀)alkenyl, (C₂-C₃₀)alkynyl, aryl(C₁-C₃₀)alkyl, aryl(C₁-C₃₀)acyl, substituted (C₁-C₃₀)alkyl, substituted (C₁-C₃₀)heteroalkyl, substituted (C₁-C₃₀)acyl, substituted (C₂-C₃₀)alkenyl, substituted (C₂-C₃₀)alkynyl, substituted aryl(C₁-C₃₀)alkyl or substituted aryl(C₁-C₃₀)acyl;
R⁴ and R⁵ is, independently for each occurrence, H, (C₁-C₄₀)alkyl, (C₁-C₄₀)heteroalkyl, (C₁-C₄₀)acyl, (C₂-C₄₀)alkenyl, (C₂-C₄₀)alkynyl, aryl(C₁-C₄₀)alkyl, aryl(C₁-C₄₀)acyl, substituted (C₁-C₄₀)alkyl, substituted (C₁-C₄₀)heteroalkyl, substituted (C₁-C₄₀)acyl, substituted (C₂-C₄₀)alkenyl, substituted (C₂-C₄₀)alkynyl, substituted aryl(C₁-C₄₀)alkyl, substituted aryl(C₁-C₄₀)acyl, (C₁-C₄₀)alkylsulfonyl or -C(NH)-NH₂;
m is, independently for each occurrence, 1, 2, 3, 4, 5, 6 or 7;
n is, independently for each occurrence, 1, 2, 3, 4 or 5;
s is, independently for each occurrence, 1, 2, 3, 4, 5, 6 or 7;
t is, independently for each occurrence, 1, 2, 3, 4, 5, 6 or 7; and
X¹, X², X³, X⁴, and X⁵ each is, independently for each occurrence, H, F, Cl, Br, I, (C₁-C₁₀)alkyl, substituted (C₁-C₁₀)alkyl, (C₂-C₁₀)alkenyl, substituted (C₂-C₁₀)alkenyl, (C₂-C₁₀)alkynyl, substituted (C₂-C₁₀)alkynyl, aryl, substituted aryl, OH, NH₂, NO₂, or CN;
provided that:
(I) when R⁴ is (C₁-C₄₀)acyl, aryl(C₁-C₄₀)acyl, substituted (C₁-C₄₀)acyl, substituted aryl(C₁-C₄₀)acyl, (C₁-C₄₀)alkylsulfonyl or -C(NH)-NH₂, then R⁵ is H, (C₁-C₄₀)alkyl, (C₁-C₄₀)heteroalkyl, (C₂-C₄₀)alkenyl, (C₂-C₄₀)alkynyl, aryl(C₁-C₄₀)alkyl, substituted (C₁-C₄₀)alkyl, substituted (C₁-C₄₀)heteroalkyl, substituted (C₂-C₄₀)alkenyl, substituted (C₂-C₄₀)alkynyl or substituted aryl(C₁-C₄₀)alkyl;
(II) when R² is (C₁-C₃₀)acyl, aryl(C₁-C₃₀)acyl, substituted (C₁-C₃₀)acyl or substituted aryl(C₁-C₃₀)acyl, then R³ is H, (C₁-C₃₀)alkyl, (C₁-C₃₀)heteroalkyl, (C₂-C₃₀)alkenyl, (C₂-C₃₀)alkynyl, aryl(C₁-C₃₀)alkyl, substituted (C₁-C₃₀)alkyl, substituted (C₁-C₃₀)heteroalkyl, substituted (C₂-C₃₀)alkenyl, substituted (C₂- C₃₀)alkynyl or substituted aryl(C₁-C₃₀)alkyl;
(III) either A³ or A⁸ or both must be present in said compound;
(IV) when A² is Cys, D-Cys, hCys, D-hCys, Pen or D-Pen, then A⁹ is Cys, D-Cys, hCys, D-hCys, Pen or D-Pen;
(V) when A² is Asp or Glu, then A⁹ is Dab, Dap, Orn or Lys;
(VI) when A⁸ is Ala or Gly, then A¹ is not Nle; and
(VII) when A¹ is deleted, then R² and R³ cannot both be H;
or a pharmaceutically acceptable salt thereof. In a preferred embodiment, the peptide is a compound of formula (I) wherein A¹ is Arg, D-Arg, hArg or D-hArg; or a pharmaceutically acceptable salt thereof.

Preferably, the active substance of the drug composition of the present invention is the peptide of formula:

Ac-Arg-c(Cys-D-Ala-His-D-Phe-Arg-Trp-Cys)-NH₂ (peptide 1)

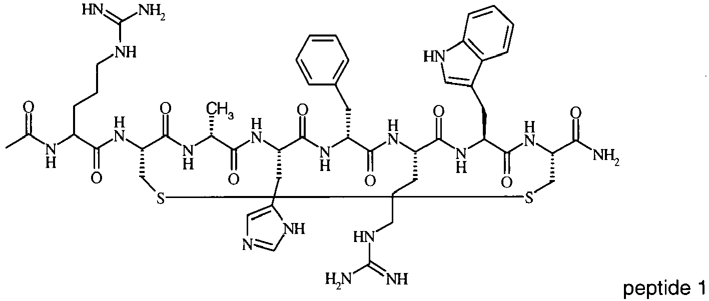

or a pharmaceutically acceptable salt thereof.

In a preferred embodiment, the peptide is a compound of formula (II): 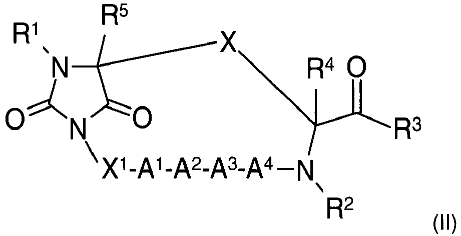 wherein the hydantoin moiety is formed from fusing the amino group of X¹, i.e.,
wherein:
X is selected from the group consisting of -CH₂-S-S-CH₂-, -C(CH₃)₂-S-S-CH₂-, -CH₂-S-S-C(CH₃)₂-, -C(CH₃)₂-S-S-C(CH₃)₂-, -(CH₂)₂-S-S-CH₂-, -CH₂-S-S-(CH₂)₂-, -(CH₂)₂-S-S-(CH₂)₂-, -C(CH₃)₂-S-S-(CH₂)₂-, -(CH₂)₂-S-S-C(CH₃)₂, -(CH₂)ₜ-C(O)-NR⁸-(CH₂)ᵣ- and -(CH₂)ᵣ-NR⁸-C(O)-(CH₂)ₜ-;
R¹ and R² each is, independently for each occurrence thereof, H, (C₁-C₁₀)alkyl or substituted (C₁-C₁₀)alkyl;
R³ is -OH or -NH₂;
R⁴ and R⁵ each is, independently for each occurrence thereof, H, (C₁-C₁₀)alkyl or substituted (C₁-C₁₀)alkyl;
X¹ is 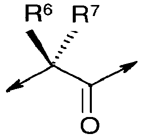
A¹ is His, 2-Pal, 3-Pal, 4-Pal, Taz, 2-Thi, 3-Thi, (X¹, X², X³, X⁴, X⁵) Phe or deleted;
A² is D-Bal, D-1-Nal, D-2-Nal, D-Phe or D-(X¹, X², X³, X⁴, X⁵) Phe;
A³ is Arg, hArg, Dab, Dap, Lys or Orn;
A⁴ is Bal, 1-Nal, 2-Nal, (X¹, X², X³, X⁴, X⁵) Phe or Trp;
R⁶ and R⁷ each is, independently for each occurrence thereof, H, (C₁-C₁₀)alkyl, (C₁-C₁₀)heteroalkyl, aryl(C₁-C₅)alkyl, substituted (C₁-C₁₀)alkyl, substituted (C₁-C₁₀)heteroalkyl or substituted aryl(C₁-C₅)alkyl or R⁶ and R⁷ may be joined together form a cyclic moiety;
R⁸ is H, (C₁-C₁₀)alkyl or substituted (C₁-C₁₀)alkyl;
r is, independently for each occurrence thereof, 1, 2, 3, 4 or 5; and
t is, independently for each occurrence thereof, 1 or 2; or
a pharmaceutically acceptable salt thereof.

Preferably, the active substance of the drug composition of the present invention is the peptide of formula:

Hydantoin(Arg-Gly))-cyclo(Cys-Glu-His-D-Phe-Arg-Trp-Cys)-NH₂ (peptide 2)

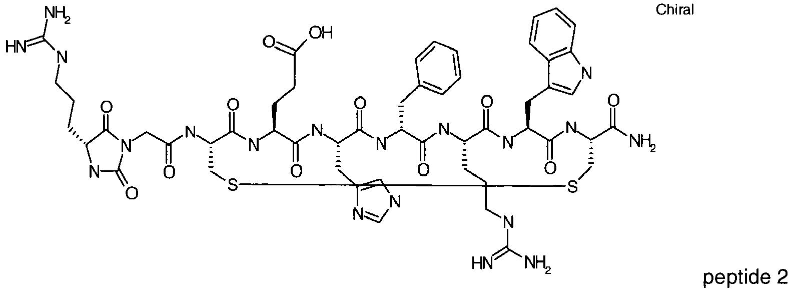

or a pharmaceutically acceptable salt thereof.

The nomenclature used to define the peptides is that typically used in the art wherein the amino group at the N-terminus appears to the left and the carboxyl group at the C-terminus appears to the right. Where the amino acid has isomeric forms, it is the L form of the amino acid that is represented unless otherwise explicitly indicated. Unless defined otherwise, all technical and scientific terms used herein have the same meaning as commonly understood by one of ordinary skill in the art to which this invention belongs. Also, all publications, patent applications, patents and other references mentioned herein are incorporated by reference. The meaning of the different symbol used above is as follows:
Abu: α-aminobutyric acid; Ac: acyl group; Acc: 1-amino-1-cyclo(C₃-C₉)alkyl carboxylic acid; A3c: 1-amino-1-cyclopropanecarboxylic acid; A4c: 1-amino-1-cyclobutanecarboxylic acid; A5c: 1-amino-1-cyclopentanecarboxylic acid; A6c: 1-amino-1-cyclohexanecarboxylic acid; Aha: 7-aminoheptanoic acid; Ahx: 6-aminohexanoic acid; Aib: α-aminoisobutyric acid; Ala or A: alanine; β-Ala: β-alanine; Apn: 5-aminopentanoic acid (HN-(CH2)₄-C(O); Arg or R: arginine; hArg: homoarginine; Asn or N: asparagine; Asp or D: aspartic acid; Bal: 3-benzothienylalanine; Bip: 4,4'-biphenylalanine, represented by the structure: 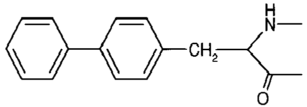
Bpa: 4-benzoylphenylalanine; 4-Br-Phe: 4-bromo-phenylalanine; Cha: β-cyclohexylalanine; hCha: homo-cyclohexylalanine; Chg: cyclohexylglycine; Cys or C: cysteine; hCys: homocysteine; Dab: 2,4-diaminobutyric acid; Dap: 2,3-diaminopropionic acid; Dip: β,β-diphenylalanine; Doc: 8-amino-3,6-dioxaoctanoic acid with the structure of: 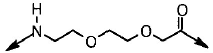
2-Fua: β-(2-furyl)-alanine; Gaba: 4-aminobutyric acid; Gln or Q: glutamine; Glu or E: glutamic acid; Gly or G: glycine; His or H: histidine; 3-Hyp: trans-3-hydroxy-L-proline, i.e., (2S, 3S)-3-hydroxypyrrolidine-2-carboxylic acid; 4-Hyp: 4-hydroxyproline, i.e., (2S, 4R)-4-hydroxypyrrolidine-2-carboxylic acid; Ile or I: isoleucine; Leu or L: leucine; hLeu: homoleucine; Lys or K: lysine; Met or M: methionine; β-hMet: β-homomethionine; 1-Nal: β-(1-naphthyl)alanine; 2-Nal: β-(2-naphthyl)alanine; Nip: nipecotic acid; Nle: norleucine; Oic: octahydroindole-2-carboxylic acid; Orn: ornithine; 2-Pal: β-(2-pyridiyl)alanine; 3-Pal: β-(3-pyridiyl)alanine; 4-Pal: β-(4-pyridiyl)alanine; Pen: penicillamine; Phe or F: phenylalanine; hPhe: homophenylalanine; Pro or P: proline; hPro: homoproline.
Ser or S: serine; Tle: tert-Leucine; Taz: β-(4-thiazolyl)alanine; 2-Thi: β-(2-thienyl)alanine; 3-Thi: β-(3-thienyl)alanine; Thr or T: threonine; Trp or W: tryptophan; Tyr or Y: tyrosine; D-(Et)Tyr has a structure of:

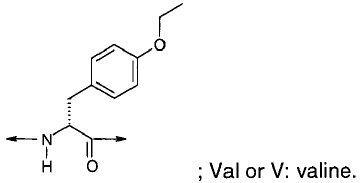

The following are definitions of terms used in this specification. The initial definition provided for a group or term herein applies to that group or term throughout the present specification, individually or as part of another group, unless otherwise indicated. Unless defined otherwise, all technical and scientific terms used herein have the same meaning as commonly understood by one of ordinary skill in the art to which this invention belongs.

The term "alkyl" refers to straight or branched chain hydrocarbon groups having 1 to 12 carbon atoms, preferably 1 to 8 carbon atoms. Lower alkyl groups, that is, alkyl groups of 1 to 4 carbon atoms, are most preferred. When a subscript is used with reference to an alkyl or other group, the subscript refers to the number of carbon atoms that the group may contain. The term "substituted alkyl" refers to an alkyl group as defined above having one, two or three substituents selected from the group consisting of halo, amino, cyano, keto (=O), -ORₐ, -SRₐ, -NRₐR_{b}, -(C=O)Rₐ, -CO₂Rₐ, -C(=O)NRₐR_{b}, -NRₐC(=O)R_{b}, -NRₐCO₂R_{b}, -OC(=O)Rₐ, -OC(=O)NRₐR_{b}, -NR_{c}C(=O)NRₐR_{b}, NRₐSO₂R_{d}, SO₂R_{d}, SO₃R_{d}, cycloalkyl, aryl, heteroaryl, or heterocycle, wherein the groups Rₐ, R_{b}, and R_{c} are selected from hydrogen, (C₁-C₆)alkyl, aryl, heteroaryl, heterocycle, cycloalkyl, or (C₁-C₆)alkyl substituted with halogen, hydroxy, methoxy, nitro, amino, cyano, -(C=O)H, -CO₂H, -(C=O)alkyl, -CO₂alkyl, -NH(alkyl), -NH(cycloalkyl), -N(alkyl)₂, carboxy, acyl, -C(=O)H, -C(=O)phenyl, -CO₂-alkyl, cycloalkyl, -(C=O)NH₂, -(C=O)NH(alkyl), -(C=O)NH(cycloalkyl), -(C=O)N(alkyl)₂, -C(=O)-(CH₂)₁₋₂NH₂, -C(=O)-(CH₂)₁₋₂NH(alkyl), -C(=O)-(CH₂)₁₋₂N(alkyl)₂, -NH-CH₂-carboxy, -NH-CH₂-CO₂-alkyl, phenyl, benzyl, phenylethyl, or phenyloxy. The group R_{d} may be selected from the same groups as Rₐ, R_{b} and R_{c} but is not hydrogen. Alternatively, the groups Rₐ and R_{b} may together form a heterocyclo or heteroaryl ring. It should be understood that when a substituted alkyl group is substituted with an aryl, cycloalkyl, heteroaryl, or heterocyclo, such rings are as defined below and thus may have one to three substituents as set forth below in the definitions for these terms. When the term "alkyl" is used as a suffix following another specifically named group, e.g., arylalkyl or heteroarylalkyl, the term defines, with more specificity, at least one of the substituents that the substituted alkyl will contain. For example, arylalkyl refers to an aryl bonded through an alkyl, or in other words, a substituted alkyl group having from 1 to 12 carbon atoms and at least one substituent that is aryl (e.g., benzyl or biphenyl). "Lower arylalkyl" refers to substituted alkyl groups having 1 to 4 carbon atoms and at least one aryl substituent.

The term "alkenyl" refers to straight or branched chain hydrocarbon groups having 2 to 12 carbon atoms and at least one double bond. Alkenyl groups of 2 to 6 carbon atoms and having one double bond are most preferred.

The term "alkynyl" refers to straight or branched chain hydrocarbon groups having 2 to 12 carbon atoms and at least one triple bond. Alkynyl groups of 2 to 6 carbon atoms and having one triple bond are most preferred. A substituted alkenyl or alkynyl will contain one, two, or three substituents as defined above for alkyl groups.

The term "alkylene" refers to bivalent straight or branched chain hydrocarbon groups having 1 to 12 carbon atoms, preferably 1 to 8 carbon atoms, e.g., {-CH₂-}ₙ, wherein n is 1 to 12, preferably 1 to 8. Lower alkylene groups, that is, alkylene groups of 1 to 4 carbon atoms, are most preferred. The terms "alkenylene" and "alkynylene" refer to bivalent radicals of alkenyl and alkynyl groups, respectively, as defined above. Substituted alkylene, alkenylene, and alkynylene groups may have substituents as defined above for substituted alkyl groups.

The term "alkoxy" refers to the group ORₑ wherein Rₑ is alkyl, substituted alkyl, alkenyl, substituted alkenyl, alkynyl, substituted alkynyl, heterocycle, or cycloalkyl. Thus, an alkoxy includes such groups as methoxy, ethoxy, cyclopropyloxy, pyrrolidinyloxy, and so forth. The term "aryloxy" refers to the groups O(aryl) or O(heteroaryl), wherein aryl and heteroaryl are as defined below.

The term "alkylthio" refers to an alkyl or substituted alkyl group as defined above bonded through one or more sulfur (-S-) atoms, e.g., -S (alkyl) or -S (alkyl-Rₐ).

The term "alkylamino" refers to an alkyl or substituted alkyl group as defined above bonded through one or more nitrogen (-NR_{f}-) groups, wherein R_{f} is hydrogen, alkyl, substituted alkyl, or cycloalkyl. The term "acyl" refers to an alkyl or substituted alkyl group as defined above bonded through one or more carbonyl {-C(=O)-} groups. When the term acyl is used in conjunction with another group, as in acylamino, this refers to the carbonyl group {-C(=O)} linked to the second named group. Thus, acylamino refers to -C(=O)NH₂, substituted acylamino refers to the group -C(=O)NRR, and acylaryl refers to -C(=O)(aryl).

The term "aminoacyl" refers to the group -NR_{f}C(=O)R_{g}, wherein R_{g} is hydrogen, alkyl, or substituted alkyl, and R_{f} is as defined above for alkylamino groups.

The term "halo" or "halogen" refers to chloro, bromo, fluoro and iodo. Unless otherwise indicated, any haloalkyl, haloalkoxy or haloalkylthio group contains one or more halo atoms which halo atoms may be the same or different.

The term "carboxy" when used alone refers to the group CO₂H. Carboxyalkyl refers to the group CO₂R, wherein R is alkyl or substituted alkyl.

The term "sulphonyl" refers to a sulphoxide group (i.e., -S(O)₁₋₂-) linked to an organic radical including an alkyl, alkenyl, alkynyl, substituted alkyl, substituted alkenyl, or substituted alkynyl group, as defined above. The organic radical to which the sulphoxide group is attached may be monovalent (e.g., -SO₂-alkyl), or bivalent (e.g., -SO₂-alkylene, etc.).

The term "cycloalkyl" refers to substituted and unsubstituted monocyclic or bicyclic hydrocarbon groups of 3 to 9 carbon atoms which are, respectively, fully saturated or partially unsaturated, including a fused aryl ring, for example, an indan. A cycloalkyl group may be substituted by one or more (such as one to three) substituents selected from alkyl, substituted alkyl, aminoalkyl, halogen, cyano, nitro, trifluoromethyl, hydroxy, alkoxy, alkylamino, sulphonyl, -SO₂(aryl), -CO₂H, -CO₂-alkyl, -C(=O)H, keto, -C(=O)-(CH₂)₁₋₂NH₂, -C(=O)-(CH₂)₁₋₂NH(alkyl), -C(=O)-(CH₂)₁₋₂N(alkyl)₂, acyl, aryl, heterocycle, heteroaryl, or another cycloalkyl ring of 3 to 7 carbon atoms. The term "cycloalkylene" refers to a cycloalkyl forming a link or spacer between two other groups, i.e., a cycloalkylene is a cycloalkyl that is bonded to at least two other groups. The term cycloalkyl includes saturated or partially unsaturated carbocyclic rings having a carbon-carbon bridge of three to four carbon atoms or having a benzene ring joined thereto. When the cycloalkyl group is substituted with a further ring, said further ring may have one to two substituents selected from Rₖ, wherein Rₖ is lower alkyl, hydroxy, lower alkoxy, amino, halogen, cyano, trifluoromethyl, trifluoromethoxy, nitro, and lower alkyl substituted with one to two hydroxy, lower alkoxy, amino, halogen, cyano, trifluoromethyl, trifluoromethoxy, and/or nitro.

The term "aryl" refers to substituted and unsubstituted phenyl, 1-naphthyl and 2-naphthyl, with phenyl being preferred. The aryl may have zero, one, two or three substituents selected from the group consisting of alkyl, substituted alkyl, alkoxy, alkylthio, halo, hydroxy, nitro, cyano, amino, trifluoromethyl, trifluoromethoxy, sulphonyl, -SO₂(aryl), -NH(alkyl), -NH(cycloalkyl), -N(alkyl)₂, carboxy, acyl, -C(=O)H, -C(=O)phenyl, -CO₂-alkyl, cycloalkyl, -(C=O)NH₂, -(C=O)NH(alkyl), -(C=O)NH(cycloalkyl), -(C=O)N(alkyl)₂, -NH-CH₂-carboxy, -NH-CH₂-CO₂-alkyl, -C(=O)-(CH₂)₁₋₂NH₂, -C(=O)-(CH₂)₁₋₂NH(alkyl), -C(=O)-(CH₂)₁₋₂N(alkyl)₂, phenyl, benzyl, phenylethyl, phenyloxy, phenylthio, heterocyclo, heteroaryl, or a (C₃-C₇)cycloalkyl ring. The term "arylene" refers to an aryl as defined above forming a link or spacer between two other groups, i.e., an arylene is an aryl that is bonded to at least two other groups. When the aryl group is substituted with a further ring, said further ring may have one to two substituents selected from Rₖ, wherein Rₖ is defined as above.

The term "heterocyclo" or "heterocycle" refers to substituted and unsubstituted non-aromatic 3- to 7-membered monocyclic groups, 7- to 11-membered bicyclic groups, and 10- to 15-membered tricyclic groups which have at least one heteroatom (O, S or N) in at least one of the rings. Each ring of the heterocyclo group containing a heteroatom can contain one or two oxygen or sulfur atoms and/or from one to four nitrogen atoms provided that the total number of heteroatoms in each ring is four or less, and further provided that the ring contains at least one carbon atom. The fused rings completing the bicyclic and tricyclic groups may contain only carbon atoms and may be saturated, partially saturated, or unsaturated. The nitrogen and sulfur atoms may optionally be oxidized and the nitrogen atoms may optionally be quatemized. The heterocyclo group may be attached at any available nitrogen or carbon atom. The heterocyclo ring may contain one, two or three substituents selected from the group consisting of halo, amino, cyano, alkyl, substituted alkyl, trifluoromethyl, trifluoromethoxy, sulphonyl, -SO₂(aryl), -NH(alkyl), -NH(cycloalkyl), -N(alkyl)₂, alkoxy, alkylthio, hydroxy, nitro, phenyl, benzyl, phenylethyl, phenyloxy, phenylthio, carboxy, -CO₂-alkyl, cycloalkyl, -C(=O)H, acyl, -(C=O)NH₂, -(C=O)NH(alkyl), -(C=O)NH(cycloalkyl),-(C=O)N(alkyl)₂, -NH-CH₂-carboxy, -NH-CH₂-CO₂-alkyl, -C(=O)-(CH₂)₁₋₂NH₂, -C(=O)-(CH₂)₁₋₂NH(alkyl), -C(=O)-(CH₂)₁₋₂N(alkyl)₂, heterocyclo, heteroaryl, a (C₃-C₇)cyloalkyl ring, keto, =N-OH, =N-O-lower alkyl, or a five or six-membered ketal, i.e., 1,3-dioxolane or 1,3-dioxane. When the heterocyclo group is substituted with a further ring, said further ring may have one to two substituents selected from Rₖ, wherein Rₖ is defined as above. Exemplary monocyclic groups include azetidinyl, pyrrolidinyl, oxetanyl, imidazolinyl, oxazolidinyl, isoxazolinyl, thiazolidinyl, isothiazolidinyl, tetrahydrofuranyl, piperidinyl, piperazinyl, 2-oxopiperazinyl, 2-oxopiperidinyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl and the like. Exemplary bicyclic heterocyclo groups include quinuclidinyl.

The term "heteroaryl" refers to substituted and unsubstituted aromatic 5- or 6-membered monocyclic groups, 9- or 10-membered bicyclic groups, and 11- to 14-membered tricyclic groups which have at least one heteroatom (O, S or N) in at least one of the rings. Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic and tricyclic groups may contain only carbon atoms and may be saturated, partially saturated, or unsaturated. The nitrogen and sulfur atoms may optionally be oxidized and the nitrogen atoms may optionally be quaternized. Heteroaryl groups which are bicyclic or tricyclic must include at least one fully aromatic ring but the other fused ring or rings may be aromatic or non-aromatic. The heteroaryl group may be attached at any available nitrogen or carbon atom of any ring. The heteroaryl ring system may contain one, two or three substituents selected from the group consisting of halo, amino, cyano, alkyl, substituted alkyl, trifluoromethyl, trifluoromethoxy, sulphonyl, -SO₂(aryl), -NH(alkyl), -NH(cycloalkyl), -N(alkyl)₂, alkoxy, alkylthio, hydroxy, nitro, phenyl, benzyl, phenylethyl, phenyloxy, phenylthio, carboxy, -CO₂-alkyl, cycloalkyl, -C(=O)H, acyl, -(C=O)NH₂, -(C=O)NH(alkyl), -(C=O)NH(cycloalkyl), -(C=O)N(alkyl)₂, -NH-CH₂-carboxy, -NH-CH₂-CO₂-alkyl, -C(=O)-(CH₂)₁₋₂NH₂, -C(=O)-(CH₂)₁₋₂NH(alkyl), -C(=O)-(CH₂)₁₋₂N(alkyl)₂, heterocylco, heteroaryl, or a (C₃-C₇)cycloalkyl ring. The heterocyclo ring may have a sulfur heteroatom that is substituted with one or more oxygen (=O) atoms. Exemplary monocyclic heteroaryl groups include pyrrolyl, pyrazolyl, pyrazolinyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, triazinyl and the like. Exemplary bicyclic heteroaryl groups include indolyl, benzothiazolyl, benzodioxolyl, benzoxaxolyl, benzothienyl, quinolinyl, tetrahydroisoquinolinyl, isoquinolinyl, benzimidazolyl, benzopyranyl, indolizinyl, benzofuranyl, chromonyl, coumarinyl, benzopyranyl, cinnolinyl, quinoxalinyl, indazolyl, pyrrolopyridyl, furopyridinyl, dihydroisoindolyl, tetrahydroquinolinyl and the like. Exemplary tricyclic heteroaryl groups include carbazolyl, benzidolyl, phenanthrollinyl, acridinyl, phenanthridinyl, xanthenyl and the like.

The peptide of the drug composition of the present invention may be in the form of a salt or as a free base.

According to one preferred embodiment, the peptide is in a salt form.

Preferably, the pharmaceutically acceptable salt of the peptide is acetate or heptanoate.

More preferably, the pharmaceutically acceptable salt of the peptide is acetate.

Advantageously, the peptide or the salt thereof is present in a concentration ranging from 0.1 to 25 % by weight relative to the total weight of the composition. More preferably, the peptide or the salt thereof is present in a concentration ranging from 0.1 to 10 % by weight, even more preferentially from 0.2 to 6 % by weight and even more preferentially still from 0.3 to 2 % by weight relative to the total weight of the composition.

According to the invention, the pharmaceutical composition comprises a water-soluble or water-dispersible gelling agent.

For the purposes of the present invention, the term "gelling agent" means an agent which, when introduced at a concentration between 0.5 and 40 % by weight in an aqueous solution makes it possible to achieve a dynamic viscosity of at least 100 cPs and preferably of at least 500 cPs, at 25° C and at a shear rate between 1 and 10 s⁻¹. This viscosity may be measured using a viscometer, for example a controlled-stress viscometer in cone-plate geometry, for instance a Haake RS1 viscometer from Thermo Electron.

Mention may be made, as gelling agents, for example, of polyols such as glycerol and propylene glycol, polyethers such as polyethylene glycols, cellulose derivatives such as microcrystalline cellulose, sodium caboxymethyl cellulose, methyl cellulose, ethyl cellulose, hydroxyethyl cellulose, hydroxypropyl cellulose and hydroxypropyl methylcellulose, mono- or polysaccharides such as sodium hyalunorate, chitosan, starch and starch derivatives, polyvinylpyrrolidone, gelatin, zinc acetate, and mixtures thereof.

The gelling agent contains one or more polyethers, one or more polyols and a mixture thereof.

According to the present invention, the gelling agent is chosen from polyethers, polyols and mixtures thereof.

More preferably, the gelling agent is chosen from polyethers and polyols.

Preferably, the gelling agent is present in a concentration ranging from 0.5 to 70 % by weight relative to the total weight of the composition. More preferably, the gelling agent is present in a concentration ranging from 10 to 50 % by weight, even more preferentially from 15 to 40 % by weight and even more preferentially still from 20 to 40 % by weight relative to the total weight of the composition.

In a preferred embodiment, the gelling agent is a polyether or a mixture of polyethers.

Preferably, the gelling agent is a polyether present in a concentration ranging from 10 to 50 % by weight relative to the total weight of the composition. More preferably, the gelling agent is a polyether and present in a concentration ranging from 15 to 40 % by weight relative to the total weight of the composition. Even more preferentially, the gelling agent is a polyether and present in a concentration ranging from 20 to 35 % by weight relative to the total weight of the composition.

In a more preferred embodiment, the gelling agent is a polyether chosen from polyethylene glycols. In another preferred embodiment, the gelling agent is the polyether PEG 400.

In a particular embodiment, the gelling agent is the polyether PEG 400 and is present in a concentration ranging from 15 to 40 % by weight relative to the total weight of the composition. More preferably, the gelling agent is the polyether PEG 400 and is present in a concentration ranging from 20 to 35 % by weight relative to the total weight of the composition.

In another preferred embodiment, the gelling agent is a polyol or a mixture of polyols.

Preferably, the gelling agent is a polyol present in a concentration ranging from 10 to 50 % by weight relative to the total weight of the composition. More preferably, the gelling agent is a polyol present in a concentration ranging from 15 to 40 % by weight relative to the total weight of the composition. Even more preferentially, the gelling agent is a polyol and present in a concentration ranging from 25 to 40 % by weight relative to the total weight of the composition.

More particularly, the gelling agent is glycerol as polyol.

In a particular embodiment, the gelling agent is glycerol as polyol and is present in a concentration ranging from 15 to 40 % by weight relative to the total weight of the composition. More preferably, the gelling agent is glycerol as polyol and is present in a concentration ranging from 25 to 40 % by weight relative to the total weight of the composition.

The composition according to the invention may also comprise one or more additives such as surfactants. These additives include fatty acids and salts thereof, polyols, polyoxyethers, poloxamers, polysorbates and polyoxyethylene fatty acid esters.

In a preferred embodiment, the composition according to the invention comprises only the peptide as active substance and the water-soluble or water-dispersible gelling agent.

Preferably, the composition according to the invention comprises only the peptide of formula Ac-Arg-c(Cys-D-Ala-His-D-Phe-Arg-Trp-Cys)-NH₂ or a pharmaceutically acceptable salt thereof and one or more polyols as gelling agent.

More preferably, the composition according to the invention comprises only the peptide of formula Ac-Arg-c(Cys-D-Ala-His-D-Phe-Arg-Trp-Cys)-NH₂ or a pharmaceutically acceptable salt thereof and glycerol as gelling agent.

Even more preferably, the composition according to the invention comprises only the peptide of formula Ac-Arg-c(Cys-D-Ala-His-D-Phe-Arg-Trp-Cys)-NH₂ or a pharmaceutically acceptable salt thereof in a concentration ranging from 0.1 to 10 % by weight relative to the total weight of the composition and glycerol as gelling agent in a concentration ranging from 15 to 40 % by weight relative to the total weight of the composition.

More preferably still, the composition according to the invention comprises only the peptide of formula Ac-Arg-c(Cys-D-Ala-His-D-Phe-Arg-Trp-Cys)-NH₂ or a pharmaceutically acceptable salt thereof in a concentration ranging from 0.3 to 2 % by weight relative to the total weight of the composition and glycerol as gelling agent in a concentration ranging from 25 to 40 % by weight relative to the total weight of the composition.

In another preferred embodiment, the composition according to the invention comprises only the peptide of formula Ac-Arg-c(Cys-D-Ala-His-D-Phe-Arg-Trp-Cys)-NH₂ or a pharmaceutically acceptable salt thereof and one or more polyethers as gelling agent.

Preferably, the composition according to the invention comprises only the peptide of formula Ac-Arg-c(Cys-D-Ala-His-D-Phe-Arg-Trp-Cys)-NH₂ or a pharmaceutically acceptable salt thereof and one or more polyethylene glycols as gelling agent.

More preferably, the composition according to the invention comprises only the peptide of formula Ac-Arg-c(Cys-D-Ala-His-D-Phe-Arg-Trp-Cys)-NH₂ or a pharmaceutically acceptable salt thereof and PEG 400 as gelling agent.

Even more preferably, the composition according to the invention comprises only the peptide of formula Ac-Arg-c(Cys-D-Ala-His-D-Phe-Arg-Trp-Cys)-NH₂ or a pharmaceutically acceptable salt thereof in a concentration ranging from 0.1 to 10 % by weight relative to the total weight of the composition and PEG 400 as gelling agent in a concentration ranging from 15 to 40 % by weight relative to the total weight of the composition.

More preferably still, the composition according to the invention comprises only the peptide of formula Ac-Arg-c(Cys-D-Ala-His-D-Phe-Arg-Trp-Cys)-NH₂ or a pharmaceutically acceptable salt thereof in a concentration ranging from 0.3 to 2 % by weight relative to the total weight of the composition and PEG 400 as gelling agent in a concentration ranging from 20 to 35 % by weight relative to the total weight of the composition.

The composition of the present invention may be prepared by mixing the peptide, the water-soluble or water-dispersible gelling agent(s) and the optional additives (if any) in water.

The pharmaceutical composition according to the invention is administered by parenteral route. In a preferred embodiment, the composition of the present invention is administered by subcutaneous route, and preferably by a subcutaneous infusion.

The pharmaceutical composition according to the invention is easily administered by the parenteral route through 27 Gauge (G) needle and more preferably through 29 G needle.

In a preferred embodiment, the composition according to the invention is formulated such that the peptide is released within a subject in need thereof for an extended period of time.

The composition according to the invention may be useful for a parenteral administration with a sustained-release of the peptide for at least 2 hours, 3 hours, 4 hours, 5 hours, 6 hours, 8 hours, 10 hours, 12 hours or 24 hours.

In a preferred embodiment, the composition according to the invention allows a sustained release for at least 2 hours. In another preferred embodiment, the composition according to the invention allows a sustained release for at least 3 hours. In another preferred embodiment, the composition according to the invention allows a sustained release for at least 6 hours. In a more preferred embodiment, the composition according to the invention allows a sustained release for at least 8 hours. In another more preferred embodiment, the composition of the present invention allows a sustained release for at least 10 hours and more preferably 12 hours.

The pharmaceutical composition according to the invention is particularly useful to treat disorders of body weight such as obesity and cachexia.

The following examples are presented to illustrate the above procedures and should not be considered as limiting the scope of the invention.

### Experimental part

### Example 1: Preparation process

Various compositions according to the present invention are prepared with peptide 1 as active ingredient according to the following process:
The gelling agent is blended (when miscible) or dissolved (when soluble) in water for injection under magnetic stirring at room temperature for 15 min at least. The peptide salt is precisely weighed and dissolved in the previously prepared composition under magnetic stirring until obtaining a clear solution. Examples of formulation compositions are reported in Table 1:

**Table 1**

| Composition # | Peptide 1 salt | Content of peptide 1 salt | Gelling agent | Content of gelling agent | WFI⁽¹⁾ content |
|---|---|---|---|---|---|
| 1 | acetate | 0.4% | PEG 400 | 32% | q.s. 100 % |
| 2 | acetate | 0.4% | PEG 400 | 22% | q.s. 100 % |
| 3 | acetate | 0.4% | glycerol | 38% | q.s. 100 % |
| 4 | heptanoate | 0.4% | PEG 400 | 32% | q.s. 100 % |

| | | | | | |
|---|---|---|---|---|---|
| ⁽¹⁾ means Water For Injection. | | | | | |

The content of peptide salt is expressed in weight percentage of product relative to the total weight of the composition. The content of gelling agent is expressed in weight percentage of agent relative to the total weight of the composition.

The four formulations of Table 1 are used in the different tests described in examples 2, 4 and 5 below.

### Example 2: Filterability testing

The filterability of compositions 1-4 was evaluated manually using a Millex GV PVDF (low protein binding Durapore^{®} PolyVinylidene DiFluoride) 0.22µm filter equipped with a 1 mL Terumo syringe.

All the formulations were shown to be filterable.

### Example 3: Solubility testing

The solubility of the peptide 1 salts in the gelling compositions of Table 1 was evaluated using HPLC analysis after filtration. Specifications of Methods 1 and 2 used for this evaluation are given in Table 2.

**Table 2**

| | HPLC Method 1 | | | | HPLC Method 2 | | | | |
|---|---|---|---|---|---|---|---|---|---|
| Column | YMC-ODS-AM 250x4.6mm 5µm | | | | YMC-ODS-AM 150x4.6mm 3µm | | | | |
| Temperature | 30° C | | | | 40° C | | | | |
| Mobile phase | A: NaH₂PO₄ (50 mM, pH 2.5) B: Acetonitrile | | | | A: H₂O + TFA (0.05 %) B: Acetonitrile + TFA (0.05 %) C: Methanol + TFA (0.05 %) | | | | |
| Flow | 1.0 mL/min | | | | 1.0 mL/min | | | | |
| UV detection | 220 nm | | | | 220 nm | | | | |
| Retention time | ∼ 31.5 min | | | | ∼ 24.6 min | | | | |
| Analysis duration | 60 min | | | | 60 min | | | | |
| Standard (100 %) | 0.6 mg/mL | | | | 0.6 mg/mL | | | | |
| Injected volume | 10 µL | | | | 10 µL | | | | |
| Dilution solvent | Acetic acid 0.1 N | | | | Acetic acid 0.1 N | | | | |
| Gradient | Time | % A | % B | Curve | | | | | |
| | 0 | 90 | 10 | / | Time | % A | % B | %C | Curve |
| | | | | | 0 | 85 | 5 | 10 | / |
| | 35 | 83 | 17 | 6 | | | | | |
| | | | | | 45 | 70 | 15 | 15 | 6 |
| | 50 | 70 | 30 | 6 | | | | | |
| | | | | | 47 | 85 | 5 | 10 | 6 |
| | 51 | 90 | 10 | 6 | | | | | |
| | | | | | 60 | 85 | 5 | 10 | 6 |
| | 60 | 90 | 10 | 6 | | | | | |

In all formulations, the peptide salts presented a solubility of at least 4 mg/mL. More precisely, the peptide 1 acetate salt showed a solubility of 100 mg/mL at least, in 32 % PEG 400 and in 38 % glycerol. The peptide 1 heptanoate salt showed a solubility of 28.1 mg/mL in 32 % PEG 400. This means that in all formulations of Table 1, the peptide salt is entirely solubilized.

### Example 4: Injectability testing

The injectability of compositions 1-4 was evaluated using a traction/compression machine which measures the injection strength during the simulated injection of the formulation from a 1 mL syringe fitted with a needle.

The maximal tolerated strength is 15 N and the most suitable needle diameter for a daily subcutaneous injection is not less than 27 Gauge.

All formulations have shown to be injectable through a 27 G needle.

### Example 5: In vitro testing

An *in vitro* testing test was developed and used to evaluate the impact of the aqueous formulations 1-4 on the peptide release profile. This test is based on the peptide diffusion from the formulation through an agarose layer into phosphate buffer saline pH 7.4 kept at 37° C.

100 mg of agarose was dissolved at 80° C in 5 mL of water under magnetic stirring. After the total agarose dissolution, the solution was cooled at 60° C.

200 µL of the formulation, which corresponds to 800 µg of peptide, were introduced in a vial. The formulation was suspended with 300 µL of the warm agarose solution heated at 60° C. The blend was then mixed and cooled at room temperature for 10 min, resulting in the first gel layer.

300 µL of 60° C warm agarose formulation-free was added on top of the first gel layer to form a second layer. The second gel layer was then cooled at room temperature for 10 min.

The second agarose layer was topped with 3.4 mL of PBS buffer kept at 37° C, as a release medium.

The vial was stoppered with a stirring rod and put in a horizontally rotating shaker in 200 rpm at 37° C.

500 µL of release medium was withdrawn for UV analysis in the upper part of the vial at the following times after the start of the test: 15 min, 30 min, 1 h, 3 h, 5 h, 9 h, 16 h, 24 h and 30 h.

Aliquots withdrawn for analysis were replaced with equal volumes of fresh PBS buffer. Each sampling was diluted at ½ in fresh PBS buffer before UV analysis.

The peptide content was measured using the UV spectrophotometer (Perkin Elmer®) at 280 nm. The peptide concentration was calculated at each time point, taking into account each peptide quantity previously withdrawn.

The concentration of the peptide released in the medium was reported as a function of the time.

The time when 50 % of the peptide is released from the formulation to the release medium (T_{50%}) was evaluated graphically, and used to compare various formulations.

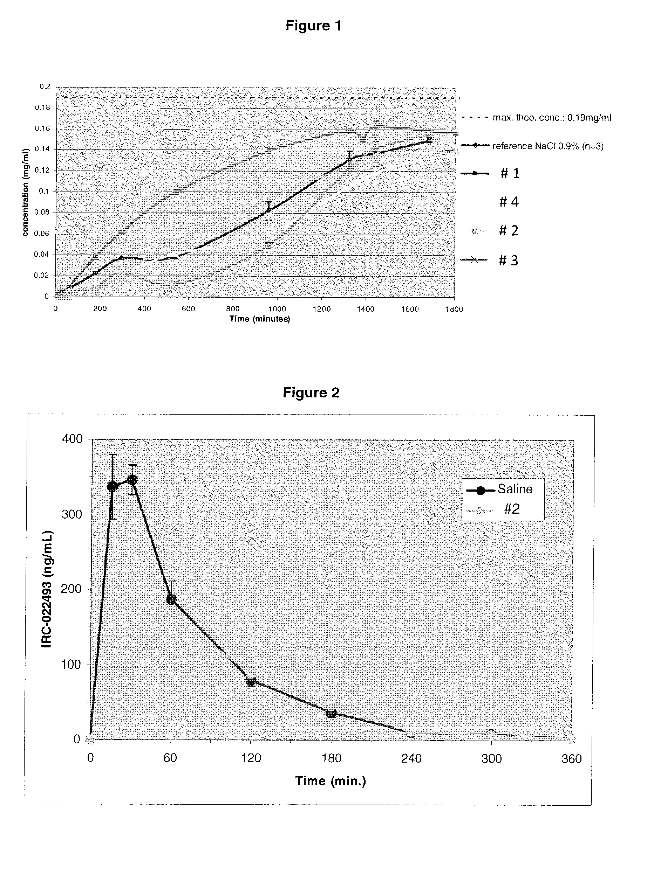

*In vitro* release profile of gelling formulations that significantly modify peptide release is given in Figure 1.

In comparison to the reference in saline, formulations 1 and 3 demonstrate a very significant peptide release slowing down. The T_{50%} values are respectively equal to 17.7 h and 19.9 h for formulations 1 and 3, versus the value for the reference in saline which is 7.8 h.

The release slowing down is also confirmed for formulations 2 and 4 with a T_{50%} being equal to 20.7 h for formulation 4 and equal to 16.2 h for formulation 2.

### Example 6: Chemical stability

Formulations manufactured with PEG 400 or glycerol as gelling agent were stored for 3 months at 40° C and 6 months at 5° C and 25° C.

A HPLC analysis was performed at various time points to evaluate the chemical stability of these compounds, using HPLC Methods 1 and 2 described in Table 2.

The selected formulations were manufactured at the maximal tolerated excipient content and
- either at the maximal estimated dose for human as follows:
   - 100 mg acetate salt of peptide 1 / mL of 30 % PEG 400 (formulation A),
   - 100 mg acetate salt of peptide 1 / mL of 32.5 % glycerol (formulation B),
- or at the maximal heptanoate solubility in 30 % PEG 400 as follows:
   - 28 mg heptanoate salt of peptide 1 / mL of 30 % PEG 400 (formulation C).

Supportive stability data of the above-mentioned formulations are respectively shown in Tables 3, 4 and 5.

**Table 3 (formulation A)**

| | T0 | Temp. | T1M | Δ (%) | T2M | Δ (%) | T3M | Δ (%) | T6M | Δ (%) |
|---|---|---|---|---|---|---|---|---|---|---|
| Estimated Content (mg/mL) | 88.1 | 40° C | | | 73.3 | -16.8 | 67.6 | -23.3 | | |
| | | 25° C | 89.7 | 1.9 | 89.2 | 1.3 | 87.6 | -0.5 | 87.5 | -0.6 |
| | | 5°C | | | | | 89.9 | 2.0 | 92.7 | 5.2 |
| Sum of Total impurities (%) | 4.3 | 40° C | | | 16.0 | 11.7 | 21.1 | 16.8 | | |
| | | 25° C | 4.9 | 0.6 | 5.6 | 1.3 | 6.1 | 1.8 | 7.9 | 3.6 |
| | | 5° C | | | | | 5.1 | 0.8 | 4.8 | 0.5 |

**Table 4 (formulation B)**

| | T0 | Temp. | T1M | Δ (%) | T2M | Δ (%) | T3M | Δ (%) | T6M | Δ (%) |
|---|---|---|---|---|---|---|---|---|---|---|
| Estimated Content (mg/mL) | 88.5 | 40°C | | | 70.7 | -20.1 | 65.6 | -25.9 | | |
| | | 25°C | 89.7 | 1.4 | 88.9 | 0.4 | 87.0 | -1.7 | 88.6 | 0.1 |
| | | 5°C | | | | | 88.3 | -0.2 | 93.0 | 5.1 |
| Sum of Total impurities (%) | 4.2 | 40° C | | | 18.7 | 14.7 | 23.6 | 19.4 | | |
| | | 25° C | 4.7 | 0.5 | 5.4 | 1.2 | 6.2 | 2.0 | 8.0 | 3.8 |
| | | 5°C | | | | | 4.7 | 0.5 | 4.7 | 0.5 |

The increase of the total impurities is moderate after 6 months at 25° C and does not exceed 5 %. After 6 months at 5° C, these formulations do not present any significant increase of the total impurities level.

**Table 5 (formulation C)**

| | T0 | Temp. | T1M | Δ (%) | T2M | Δ (%) | T3M | Δ (%) | T6M | Δ (%) |
|---|---|---|---|---|---|---|---|---|---|---|
| Estimated Content (mg/mL) | 27.5 | 40° C | | | 23.6 | -14.1 | 21.7 | -21.2 | | |
| | | 25° C | 26.3 | -4.3 | 28.7 | 4.5 | 28.6 | 4.2 | | |
| | | 5°C | | | | | 23.1 | -16.0 | | |
| Sum of Total impurities (%) | 5.6 | 40° C | | | 14.4 | 8.8 | 23.8 | 18.2 | | |
| | | 25° C | 6.9 | 1.3 | 7.3 | 1.7 | 9.0 | 3.4 | 12.9 | 7.3 |
| | | 5°C | | | | | 7.0 | 1.4 | 8.5 | 2.9 |

Formulation C also presents a moderate increase of the total impurities after 3 months at 25° C which does not exceed 5 %.

After 6 months at 5° C, this formulation presents a moderate increase of the total impurities level; this increase of total impurities tends to be quite higher than the one obtained for the previous formulations with acetate salt, but still remains lower than 5 %.

The selected formulations were then manufactured to evaluate the possibility to inject at lower doses:
- 10 mg acetate salt of peptide 1 / mL of 30 % PEG 400 (formulation D),
- 7.5 mg heptanoate salt of peptide 1 / mL of 22.5 % PEG 400 (formulation E).

Supportive stability data of the above-mentioned formulations are respectively shown in Tables 6 and 7:

**Table 6 (formulation D)**

| | T0 | Temp. | T1M | Δ (%) | T3M | Δ (%) |
|---|---|---|---|---|---|---|
| Estimated Content (mg/mL) | 10.0 | 40°C | 9.9 | -0.5 | 9.1 | -9.1 |
| | | 25°C | | | 10.1 | 1.3 |
| Sum of Total impurities (%) | 2.4 | 40° C | 4.7 | 2.3 | 6.9 | 4.5 |
| | | 25° C | | | 3.0 | 0.6 |

**Table 7 (formulation E)**

| | T0 | Temp. | T3M | Δ (%) |
|---|---|---|---|---|
| Estimated Content (mg/mL) | 6.3 | 40°C | 5.5 | -12.8 |
| | | 25° C | 6.3 | 0.5 |
| Sum of total impurities (%) | 7.7 | 40°C | 10.6 | 2.9 |
| | | 25° C | 7.1 | 0.6 |

After 3 months at 40° C, these formulations present a moderate increase of the total impurities tending to 5 % and do not present any significant increase of the total impurities level after 3 months at 25° C.

### Example 7: In vivo testing

PK profiles of the formulations according to the invention were evaluated in rats. Eight rats divided in two groups of four were used per formulation. Each animal received a subcutaneous (SC) injection at a dose of 0.5 mg/kg, and then blood sampling was performed via a jugular catheter at different time points alternately on each group. Plasma concentrations were determined by LC-MS analysis. PK parameters were calculated by a WinNonLin analysis.

PK values were compared to the one obtained after the injection of the peptide in a saline solution under the same conditions. PK profile is presented in Figure 2 (PK profiles in rats - 450 nmole/kg, 0.5 mg/kg, SC).

PK parameters of the formulation 2 and saline reference are shown in Table 8.

**Table 8**

| | Reference | Formulation 2 |
|---|---|---|
| Tₘₐₓ (min) | 30 | 60 |
| Cₘₐₓ (ng/ml) | 346 | 164 |
| T_{1/2} (min) | 49.4 | 52.4 |
| MRT (min) | 74.5 | 102 |
| AUC (min.ng/ml) | 29.6 | 20.1 |

Cₘₐₓ: maximum plasma concentration of the drug appearing in the PK profile; AUC: Area Under the Curve; MRT: Medium Residence Time; Tₘₐₓ: time corresponding to the Cₘₐₓ value; T_{1/2}: half-life.

These results confirm a significant decrease of Cₘₐₓ together with an increase of Tₘₐₓ by a factor 2, for a concentration of 22 % of PEG 400. Formulation 2 appears to be efficient enough to significantly reduce the Cₘₐₓ and increase the Tₘₐₓ in rats.

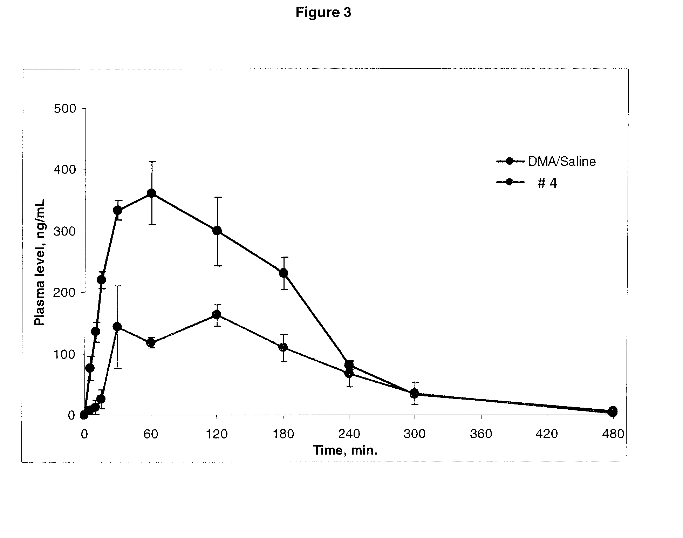

The PK profile and parameters of formulation 4 are presented in Figure 3 (PK profiles in rats - 450 nmole/kg, 0.5 mg/kg, SC) and Table 9 (PK parameters of the formulation 4 and DMA/saline reference) respectively.

**Table 9**

| | Reference | Formulation 4 |
|---|---|---|
| Tₘₐₓ (min) | 60 | 120 |
| Cₘₐₓ (ng/ml) | 362 | 163 |
| T_{1/2} (min) | 186 | 189 |
| MRT (min) | 145 | 191 |
| AUC (min.ng/ml) | 71.7 | 35.9 |

Similarly to formulation 2, formulation 4 presents a Cₘₐₓ decrease by more than a factor 2 and a Tₘₐₓ increase by a factor 2.

## Claims

1. Aqueous pharmaceutical composition comprising:
- a peptide as the active ingredient, and
- one or more water-soluble or water-dispersible gelling agents.

2. Composition according to claim 1, wherein the peptide is selected from a ligand of one or more of melanocortin receptors (MC-R).

3. Composition according to claim 1, wherein the peptide is a ligand of melanocortin MC4 receptor.

4. Composition according to any one of the preceding claims, wherein the peptide is Ac-Arg-c(Cys-D-Ala-His-D-Phe-Arg-Trp-Cys)-NH₂ or a pharmaceutically acceptable salt thereof.

5. Composition according to any one of the preceding claims, wherein the peptide is in a salt form.

6. Composition according to any one of the preceding claims, wherein the pharmaceutically acceptable salt of the peptide is acetate or heptanoate, and preferably acetate.

7. Composition according to any one of the preceding claims, wherein the peptide or the salt thereof is present in a concentration ranging from 0.1 % to 25 % by weight, preferably from 0.1 % to 10 % by weight relative to the total weight of the composition.

8. Composition according to any one of the preceding claims, wherein the gelling agent is chosen from polyethers and polyols.

9. Composition according to any one of the preceding claims, wherein the gelling agent is present in a concentration ranging from 0.5 to 70 % by weight, preferably from 10 to 50 % by weight relative to the total weight of the composition.

10. Composition according to claim 8, wherein the polyether is chosen from polyethylene glycols and preferably is PEG 400.

11. Composition according to claim 10, wherein the polyether is PEG 400 present in a concentration ranging from 15 to 40 % by weight and preferably from 20 to 35 % by weight relative to the total weight of the composition.

12. Composition according to claim 8, wherein the polyol is glycerol.

13. Composition according to claim 12, wherein the polyol is glycerol present in a concentration ranging from 15 to 40 % by weight and preferably from 25 to 40 % by weight relative to the total weight of the composition.

14. Composition according to any one of the preceding claims, wherein the peptide is released within a subject in need thereof for an extended period of time.

15. Composition according to claim 8, wherein said release of said compound extends for at least 2 hours, preferably for at least 6 hours, more preferably for at least 8 hours, even more preferably for at least 10 hours, and better still for at least 12 hours.
